# EUROPEAN PATENT SPECIFICATION

(11) **EP 0 597 224 B1**
(45) Date of publication and mention of the grant of the patent: **29.12.1999**
(21) Application number: 93115472.8
(22) Date of filing: 24.09.1993
(51) Int. Cl.: D01F 8/04, D01F 8/06, D01F 8/10, D01F 8/12, D01F 8/14, D01F 8/16, D04H 3/00, A47K 10/02, A47L 13/16, A61F 13/15

(54) **Hydrophilic, multicomponent polymeric strands and nonwoven fabrics made therewith**
Hydrophile, polymere Mehrkomponentenfäden und nichtgewebte Stoffe daraus
Fils hydrophiles polymères à plusieurs composants et non-tissés de ceux-ci

(30) Priority: 12.11.1992 US 974554
(43) Date of publication of application: 18.05.1994
(73) Proprietor: KIMBERLY-CLARK WORLDWIDE, INC., Neenah, Wisconsin 54956 (US)
(72) Inventor: Swee Chye Yeo, Richard, Dunwoody, Georgia 30338 (US); Cosgrove Creagan, Christopher, Marietta, Georgia 30062 (US)
(74) Representative: Grünecker, Kinkeldey, Stockmair & Schwanhäusser Anwaltssozietät

(56) References cited:
- EP-A- 0 311 860
- EP-A- 0 340 763
- EP-A- 0 434 029

## Description

This invention generally relates to polymeric fibers and filaments and products such as nonwoven fabrics made with polymeric fibers and filaments. More particularly, this invention relates to wettable polymeric fibers and filaments and nonwoven fabrics made with such fibers and filaments.

Polymeric fibers and filaments are used to make a variety of products including yarns, carpets, woven fabrics, and nonwoven fabrics. As used herein, polymeric fibers and filaments are referred to generically as polymeric strands. Filaments mean continuous strands of material and fibers mean cut or discontinuous strands having a definite length.

Some products made with polymeric strands must be wettable with water or aqueous solutions. In other words, some products made with polymeric strands must be hydrophilic. Nonwoven fabrics are particularly suited for making hydrophilic products. Such products include towels, wipes, and absorbent personal care products including infant care items such as diapers, child care items such as training pants, feminine care items such as sanitary napkins, and adult care items such as incontinence products. Typical polymers used to make wettable nonwoven fabric include linear polycondensates such as polyamides, polyesters and polyurethanes and crystalline polyolefins such as polyethylene, polypropylene, and copolymers of ethylene and propylene. However, such polymers are naturally hydrophobic and must be treated to become hydrophilic.

Methods for treating hydrophobic polymeric strands and materials made therewith include solution coating of wetting agents, internal incorporation of wetting agents, and plasma treatment. These methods are effective but suffer some drawbacks. For example, wetting agents, whether in a surface coating or internally incorporated into the polymer, are fugitive and wash-off of the material after one or more wettings. Once the surface agent has been washed-off the polymer, the polymer becomes hydrophobic again and repels water. Plasma treatment is slow and costly and thus commercially impractical.

Naturally hydrophilic polymers for making polymeric strands are known. These polymers do not require any treatment to become wettable but suffer from some disadvantages. For example, U.S.-A-4,163,078; US-A-4,257,999; and US-A-4,810,449 each disclose hydrophilic filaments or fibers made by solution spinning acrylonitrile copolymers. Solution spinning is relatively costly and requires the use of organic solvents which are a potential environmental hazard. Melt-extruded, hydrophilic fibers for making fibers and filaments are known, but are uncommon and expensive and thus are not normally commercially feasible.

EP-A-340763, EP-A-311860 and EP-A-434029 disclose core/sheath fibers which have a hydrophilic component which constitutes at least part of the peripheral surface of the strands.

Therefore, there is a need for low-cost, permanently hydrophilic polymeric fibers and filaments and products such as nonwovens made therewith.

Accordingly, the object of the present invention is to provide improved polymeric strands and products made therewith such as nonwovens and absorbent articles, particulary permanently hydrophilic polymeric strands and products made therewith and permanently hydrophilic polymeric strands and products made therewith without the use of surfactant treatments or other conventional treatment methods and particulary permanently hydrophilic polymeric strands and products made therewith without the use of wet spinning methods especially permanently hydrophilic polymeric strands. This object has been solved with a melt-extruded multicomponent polymeric strand including a first melt-extrudable polymeric component and a second melt-extrudable, hydrophilic polymeric component, the multicomponent strand having a cross-section, a length, and a peripheral surface, the first and second components being arranged in distinct zones across the cross-section of the multicomponent strand and extending continuously along the length of the multicomponent strand, the second component constituting at least a portion of the peripheral surface of the multicomponent strand continuously along the length of the multicomponent strand, **characterised in that** the second component has a critical surface tension at 20 ° C greater than 55 dyne/cm.

Preferably the first and second components are arranged in a sheath/core configuration, the first component forming the core and the second component forming the sheath.

Preferably the second component has a critical surface tension at 20 ° C greater than 65 dyne/cm.

Because the polymeric strand of the present invention includes a hydrophilic polymeric component, no surfactant treatment or plasma treatment is necessary to make the strand hydrophilic. Without having to use such conventional treatments, the strand of the present invention can be made more economically. In addition, because the polymeric strand of the present invention is melt-extruded and not solution spun, the strand of the present invention is made without the use of organic solvents and therefore is more economical and safe for the environment than solution spun strands.

The polymeric strand of the present invention may be arranged in a side-by-side configuration or in a sheath/core configuration; however, the first and second components are preferably arranged in a sheath/core configuration, the first component forming the core and the second component forming the sheath so that the second hydrophilic component forms the peripheral surface of the multicomponent strand. With the second hydrophilic component forming the peripheral surface of the multicomponent strand, the multicomponent strand is completely hydrophilic.

The melt-extrudable, first component of the multicomponent polymeric strand of the present invention can be hydrophobic because it is the second component that renders the strand hydrophilic. Suitable polymers for the first component are melt-extrudable and include linear polycondensates and crystalline polyolefins. The first component preferably has a considerably lower cost than the second component so that the overall cost of the strand is low. Particularly suitable polymers for the first component include polypropylene, polyethylene, copolymers of ethylene and propylene, polyethylene terephthalate, and polyamides.

The second component is melt-extrudable and hydrophilic. As used herein, hydrophilic means wettable with water or an aqueous solution. Suitable polymers for the second component are those on whose surface water or an aqueous solution will wet-out. Generally, to be wettable, the polymer must have a critical surface tension equal to or greater than the surface tension of the liquid. Preferably, the second component of the present invention has a critical surface tension at 20°C greater than about 65 dyne/cm. Preferably, the second component comprises a block copolymer of nylon 6 and polyethylene oxide diamine. Other suitable polymers for the second component are ethylene acrylic acid and its neutralized salts.

Preferably, the first component of the polymeric strand of the present invention is present in an amount from about 50 to 95% by weight of the strand and the second component is present in an amount from about 50 to about 5% of the strand. More preferably, the first component of the polymeric strand of the present invention is present in an amount from about 50 to 85% by weight of the strand and the second component is present in an amount from about 50 to about 15% of the strand.

The nonwoven fabric of the present invention comprises the above-described melt-extruded multicomponent polymeric strands and may be made by conventional techniques for making nonwovens such as melt spinning followed by bonding. The absorbent articles of the present invention include a fluid handling layer of the above described nonwoven fabric.

Still further objects and the broad scope of applicability of the present invention will become apparent to those of skill in the art from the details given hereafter. However, it should be understood that the detailed description of the preferred embodiments of the present invention is only given by way of illustration because various changes and modifications well within the scope of the invention should become apparent to those of skill in the art in view of the following detailed description.

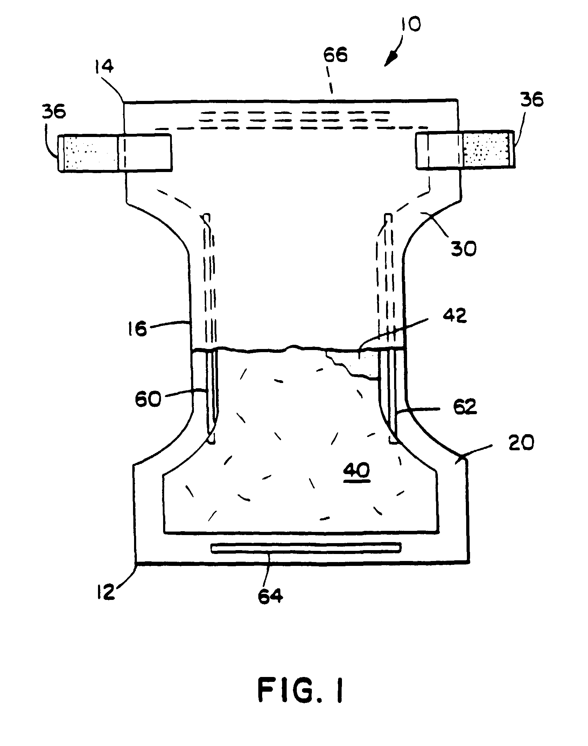

Figure 1 is a partial plan view of an absorbent diaper-type article made according to a preferred embodiment of the present invention. Portions of some layers of the article have been removed to expose the interior of the article.

It is preferred that the first component comprises a polymer selected from the group consisting of polypropylene, polyethylene, copolymers of ethylene and propylene, polyethylene terephthalate, and polyamides and the second component comprises a block copolymer of nylon 6 and polyethylene oxide diamine.

These can be arranged so that the first and second components are arranged in a sheath/core configuration, the first component forming the core and the second component forming the sheath.

In this respect, it is preferred that the first component is present in an amount from 50 to 95% by weight of the strand and the second component is present in an amount from 50 to 5% of the strand, more preferably the first component is present in an amount from 50 to 85% by weight of the strand and the second component is present in an amount from 50 to 15% of the strand.

The present invention further provides a non-woven fabric comprising melt-extruded multicomponent polymeric strands including a first melt-extrudable polymeric component and a second melt-extrudable, hydrophilic polymeric component, the multicomponent strands having a cross-section, a length, and a peripheral surface, the first and second components being arranged in distinct zones across the cross-section of the multicomponent strands and extending continuously along the length of the multicomponent strands, the second component constituting at least a portion of the peripheral surface of the multicomponent strands continuously along the length of the multicomponent strands, **characterised in that** the second component has a critical surface tension at 20°C greater than 55 dyne/cm.

Preferably the first and second components are arranged in a sheath/core configuration, the first component forming the core and the second component forming the sheath.

Preferably the second component has a critical surface tension at 20°C greater than 65 dyne/cm.

The second component comprises preferably a block copolymer of nylon 6 and polyethylene oxide diamine or ethylene acrylic acid or neutralised salt of ethylene acrylic acid.

Preferably the first component is hydrophobic and can be selected from the group consisting of linear polycondensates and crystalline polyolefins, it preferably comprises a polymer selected from the group consisting of polypropylene, polyethylene, copolymers of ethylene and propylene, polyethylene terephthalate, and polyamides.

It is preferred that the first component comprises a polymer selected from the group consisting of polypropylene, polyethylene, copolymers of ethylene and propylene, polyethylene terephthalate and polyamides and the second component comprises a block copolymer of nylon 6 and polyethylene oxide diamine.

Further preferred is an arrangement wherein the first and second components are arranged in a sheath/core configuration, the first component forming the core and the second component forming the sheath.

The first component is preferably present in an amount from 50 to 95% by weight of the strands and the second component is preferably present in an amount from 50 to 5% of the strands, more preferably the first component is present in an amount from 50 to 85% by weight of the strands and the second component is present in an amount from 50 to 15% of the strands.

Also for the core/sheath configuration it is preferred that the first component is present in an amount from 50 to 95% by weight of the strands and the second component is present in an amount from 50 to 5% of the strands, more preferably the first component is present in an amount from 50 to 85% by weight of the strands and the second component is present in an amount from 50 to 15% of the strands.

The present invention further provides an absorbent article comprising a fluid handling layer of nonwoven fabric comprising melt-extruded multicomponent polymeric strands including a first melt-extrudable polymeric component and a second melt-extrudable, hydrophilic polymeric component, the multicomponent strands having a cross-section, a length, and a peripheral surface, the first and second components being arranged in distinct zones across the cross-section of the multicomponent strands and extending continuously along the length of the multicomponent strands, the second component constituting at least a portion of the peripheral surface of the multicomponent strands, **characterised in that** the second component has a critical surface tension at 20°C greater than 55 dynes/cm.

Preferably the first and second components are arranged in a sheath/core configuration, the first component forming the core and the second component forming the sheath.

Preferably the second component has a critical surface tension at 20°C greater than 65 dyne/cm.

Preferably the second component comprises a block copolymer of nylon 6 and polyethylene oxide diamine or ethylene acrylic acid or neutralised salt of ethylene acrylic acid.

Preferably the first component is hydrophobic and more preferably is selected from the group consisting of linear polycondensates and crystalline polyolefins, in particular comprises a polymer selected from the group consisting of polypropylene, polyethylene, copolymers of ethylene and propylene, polyethylene terephthalate, and polyamides.

It is preferred that the first component comprises a polymer selected from the group consisting of polypropylene, polyethylene, copolymers of ethylene and propylene, polyethylene terephthalate, and polyamides and the second component comprises a block copolymer of nylon 6 and polyethylene oxide diamine.

In this respect it is preferred that the first and second components are arranged in a sheath/core configuration, the first component forming the core and the second component forming the sheath.

Preferably the first component is present in an amount from 50 to 95% by weight of the strands and the second component is present in an amount from 50 to 5% of the strands, more preferably the first component is present in an amount from 50 to 85% by weight of the strands and the second component is present in an amount from 50 to 15% of the strands.

Also in the core/sheath configuration it is preferred that the first component is present in an amount from 50 to 95% by weight of the strands and the second component is present in an amount from 50 to 5% of the strands, more preferably the first component is present in an amount from 50 to 85% by weight of the strands and the second component is present in an amount from 50 to 15% of the strands.

The absorbent article may be an adult incontinence product, an infant diaper, a wipe, a towel, a training pant, or a feminine care absorbent product.

The present invention provides a melt-extruded, multicomponent, hydrophilic polymeric strand, a nonwoven fabric made with such polymeric strands, and absorbent articles made with such nonwoven fabric. The nonwoven fabric of the present invention is suitable to make absorbent articles including towels, wipes, and absorbent personal care products including infant care items such as diapers, child care items such as training pants, feminine care items such as sanitary napkins, and adult care items such as incontinence products. The hydrophilic nonwoven fabric of the present invention is particularly suitable for making the fluid handling layers of a disposable diaper such as the liner, surge, transfer and distribution layers of a disposable diaper.

Generally described, the melt-extruded, multicomponent polymeric strand of the present invention includes a first melt-extrudable polymeric component and a second melt-extrudable, hydrophilic polymeric component. The first and second components are arranged in distinct zones across the cross-section of the multicomponent strand and extend continuously along the length of the multicomponent strand. The second component constitutes at least a portion of the peripheral surface of the multicomponent strand continuously along the length of the multicomponent strand.

The multicomponent polymeric strand of the present invention is preferably arranged so that the first and second components are in a sheath/core configuration with the first component forming the core and the second component forming the sheath. The multicomponent polymeric strand of the present invention can also be arranged in a side-by-side configuration; however, the sheath/core configuration tends to result in a more hydrophilic strand because the hydrophilic second component forms the peripheral surface of the strand. The peripheral surface is then hydrophilic and the first component is masked.

The first component of the polymeric strand can be hydrophobic and preferably is a low-cost polymer so that the overall cost of the multicomponent strand is less than if the multicomponent strand was made entirely of the hydrophilic second component. The first component should be melt-extrudable. Melt-extrudable means that the polymer is thermally stable at the melting temperature of the polymer. In other words, a melt-extrudable polymer does not appreciably decompose or cross-link at or below the melting temperature of the polymer.

Suitable melt-extrudable multicomponent polymers for the first component include linear polycondensates and crystalline polyolefins. Preferably, the first component has a first melt viscosity which is higher than the melt viscosity of the second component. Typically, when the melt viscosity of the first component is higher than the melt viscosity of the second component, the multicomponent strand is more easily and consistently melt-spun in the sheath/core configuration. More particularly, suitable polymers for the first component include polypropylene, polyethylene, copolymers of ethylene and propylene, polyethylene terephthalate, and polyamides. ESCORENE^{®} PP 3445 polypropylene available from Exxon of Houston, Texas is particularly preferred.

The second component of the multicomponent polymeric strand of the present invention should be melt-extrudable and hydrophilic. As explained above, hydrophilic is used herein to mean wettable with water or an aqueous solution. Suitable polymers for the second component are those on whose surface water or an aqueous solution will wet-out. Generally, to be wettable, the polymeric component must have a critical surface tension greater than or equal to the surface tension of the liquid. The second component of the multicomponent polymeric strand of the present invention has a critical surface tension greater than about 55 dyne/cm, and more preferably has a critical surface tension at 20°C greater than about 65 dyne/cm. The second component preferably includes a block copolymer of nylon 6 and polyethylene oxide diamine. Such a block copolymer is available from Allied Signal, Inc. of Petersburg, Virginia under the mark HYDROFIL^{®}. Other suitable polymers for the second component are ethylene acrylic acid and its neutralized salts. Such polymers are available from Allied Signal, Inc. under the mark ACLYN^{®}.

The first component of the multicomponent polymeric strand of the present invention is preferably present in an amount from about 50 to about 95% by weight of the strand and the second component is preferably present in an amount from about 50 to about 5% of the strand. More preferably, the first component of the polymeric strand of the present invention is present in an amount from about 50 to 85% by weight of the strand and the second component is present in an amount from about 50 to about 15% of the strand. Most preferably, the first component includes polypropylene and the second component includes a block copolymer of nylon 6 and polyethylene oxide diamine, the first and second components being present in the foregoing amounts.

The multicomponent polymeric strand of the present invention can be made by conventional melt-extrusion techniques such as melt-spinning. A preferred method of melt-spinning the multicomponent polymeric strands of the present invention and making a nonwoven fabric therewith is disclosed in U.S.-A-4,340,563 to Appel et al.. Although U.S.-A-4,340,563 discloses only single polymeric component filaments, methods for modifying that disclosure to produce multicomponent filaments are well-known to those of skill in the art. Other suitable processes for making the multicomponent polymeric strands of the present invention are disclosed in U.S.-A-3,423,266 to Davies et al., U.S.-A-3,595,731 to Davies et al., and U.S.-A-3,802,817 to Matsuki et al..

Generally described, the melt-spinning apparatus disclosed in U.S. -A-4,340,563 includes an extruder for extruding polymeric material through a spin box. The spin box includes a conventional spinneret for making polymeric filaments. The filaments are spun through the spinneret which has one or more rows of openings and formed into a curtain of filaments. The curtain of filaments is directed into a quench chamber extending downwardly from the spin box. Air is introduced into the quench chamber through an inlet port and contacts the filaments. A portion of the quench air is directed through the filament curtain and exhausted through an outlet port opposite the inlet port. The remaining portion of the quench air is directed downwardly through the quench chamber through a smoothly narrowing lower end of the quenching chamber into a nozzle wherein the quench air achieves a higher velocity. The drawing nozzle has a full machine width and is formed by a stationary wall and a moveable wall. The moveable wall moves relative to the stationery wall to control the speed of the air through the nozzle. The quench air directs the curtain of filaments out of the quenching chamber through the nozzle and deposits the filaments on a moving foraminous surface to form a nonwoven web. The nonwoven web can then be bonded by conventional means such as through-air bonding by contacting the nonwoven web with heated air or thermal point bonding.

For the present invention, multicomponent filaments can be made with the foregoing method disclosed in U.S.-A-4,340,563 by incorporating a conventional extrusion system and spinneret for making multicomponent filaments. Such extrusion systems and spinnerets are well-known to those of ordinary skill in the art.

Through-air bonding and thermal point bonding methods are well-known to those of skill in the art. Generally described, a through-air bonder includes a perforated roll which receives the fabric web and a hood surrounding the perforated roll. Air having a temperature sufficient to soften the second component of the filaments and form bonds between the filaments is directed from the hood, through the fabric web, and into the perforated roll. A thermal point bonder includes a pair of adjacent rolls, one having an array of raised points. One or both of the rolls are heated and the fabric web is passed through the nip between the rolls. The raised points compress, soften and bond the web forming an array of bond points across the web. Thermal point bonding can be conducted in accordance with U.S.-A-3,855,046.

The following examples are designed to illustrate particular embodiments of the present invention made according to the process disclosed in U.S.-A-4,340,563 using conventional bicomponent melt-spinning techniques and teach one of ordinary skill in the art how to carry out the present invention.

### EXAMPLES 1-6

Six nonwoven fabrics comprising bicomponent polymeric filaments were made according to the process disclosed in U.S. -A-4,340,563 and conventional bicomponent melt-spinning techniques. The process parameters for Examples 1-6 are set forth in Table 1 along with properties of the resulting nonwoven fabrics.

For each of the Examples 1-6, the first component comprised ESCORENE^{®} PP 3445 polypropylene available from Exxon of Houston, Texas and the second component comprised HYDROFIL^{®} LCFX copolymer of nylon 6 and polyethylene oxide diamine available from Allied Signal, Inc. of Petersburg, Virginia. At 250°C, the HYDROFIL^{®} LCFX copolymer had a melt flow rate of 61.6 grams per 10 minutes and a melt density of 0.95 grams per cc, and the ESCORENE^{®} PP 3445 polypropylene had a melt flow rate of 54.2 grams/10 minutes and a melt density of 0.73 grams/cc. The Hydrofil^{®} LCFX copolymer had a critical surface tension of about 69 dyne/cm based on static contact angle measurement with water at 20°C.

For Examples 1-6, the quench zone had a length of 96,52 cm (38 inches) and the quench outlet nozzle had a length of 101,6 cm (40 inches). The basis weight of each of the fabrics from Examples 1-6 was 1 oz. per square yard. The filaments in Examples 1-5 were arranged in a sheath/core (S/C) configuration and the filaments in Example 6 had a side-by-side (S/S) configuration.

Samples of fabric from Examples 1-6 were tested for absorbency according to the penetration rate test and the run-off test and the results are shown in Table 1.

The process for the penetration rate test is as follows:

A (5x6 inch) 12,7 x 15,24 cm test sample is placed on a (5x6 inch) 12,7 x 15,24 cm diaper absorbent pad having a fluff and superabsorbent polymer mixture and then a Lucite plate is placed on the test material. The Lucite plate has dimensions of 12,7 x 15,24 x 0,635 cm (5x6x1/4 inch) with a (3/4 inch) 1,905 cm diameter hole at the center. Extra weight is added onto the Lucite plate to produce a pressure of (0.15 psi) 1,034 KPa on the test material. 50 cc of synthetic urine is poured through the hole of the Lucite plate allowing the fluid to fill but not overflow the hole. After 3 minutes, another 26 cc of synthetic urine is poured through the hole again at a rate to fill but not overflow the hole. The time from the second application of the urine until all the fluid has passed through the material is recorded as the penetration rate. A shorter time means a faster penetration rate.

The fluid run-off test method is as follows:

A (3x6 inch) 7,62 x 15,24 cm test sample is placed on a (3x4 inch) 7,62 x 10,16 cm diaper absorbent pad which can absorb at least 6 milliliters of test fluid and both materials are placed on a 30° inclined plane. A polyethylene film is placed loosely on the test sample and is 2.54 cm (1 inch) away from the point where the test fluid contacts the sample. 60 cc of synthetic urine test fluid is then poured from a separatory funnel with the bottom of the funnel 1 centimeter from the top of the test sample. A beaker is placed under the collecting tube of the inclined plane to collect the test fluid run-off from the test sample. The weight of the fluid run-off is recorded and the procedure is repeated three more times. The absorbent pad is replaced after each fluid insult. The total weight of fluid run-off for the 4 insults is recorded. A lower weight indicates a better penetration performance.

The penetration rate and run-off tests were performed 5 times and the averages of those 5 tests are shown in Table 1. As can be seen from the data in Table 1, the fabric samples from Examples 1-6 were highly wettable and absorbent with synthetic urine. Synthetic urine has a surface tension of about 56 dyne/cm at 20°C. Example 5 shows that filaments in a sheath/core arrangement having the hydrophilic second component present in an amount of only 10% by weight are hydrophilic. It was observed, however, that filaments arranged in a side-by-side configuration having the second component present in an amount less than 50% by weight were considerably less wettable than filaments having a side-by-side configuration with the second component present in an amount of 50% by weight or greater or filaments having a sheath/core configuration.

**TABLE 1**

| | EXAMPLE 1 | EXAMPLE 2 | EXAMPLE 3 | EXAMPLE 4 | EXAMPLE 5 | EXAMPLE 6 |
|---|---|---|---|---|---|---|
| Configuration | S/C | S/C | S/C | S/C | S/C | S/S |
| | | | | | | |
| Weight % of Second Component | 40 | 30 | 20 | 20 | 10 | 50 |
| | | | | | | |
| 1st Component Melt Temp (°F) °C | (498) 259 | (499) 259,4 | (499) 259,4 | (463) 239,4 | (469) 242,8 | (458) 236,7 |
| | | | | | | |
| 2nd Component Melt Temp (°F) °C | (533) 278 | (537) 280,5 | (540) 282,2 | (525) 273,9 | (534) 278,9 | (505) 262,8 |
| | | | | | | |
| Quench Air SCFM/In | 35 | 30 | 35 | 35 | 35 | 40 |
| | | | | | | |
| Quench Air Temp (°F) °C | (50) 10 | (50) 10 | (50) 10 | (50) 10 | (51) 10,5 | (50) 10 |
| | | | | | | |
| Quench Duct Pressure (in H₂O) | 22 | 22 | 26 | 30 | 21 | 26 |
| | | | | | | |
| Total Throughput Grams/hole/min | 1.0 | 1.0 | 1.0 | 1.0 | 1.0 | 0.75 |
| | | | | | | |
| Denier | 9.2 | 10.1 | 6.1 | 4.9 | 6.6 | 4.9 |
| | | | | | | |
| Penetration Rate (sec) | 47.3 | 38.8 | 48.8 | 48.3 | 46.7 | 37.3 |
| | | | | | | |
| Run-off (g) | 0.00 | 0.00 | 0.00 | 0.00 | 0.00 | 0.07 |

Turning to Figure 1, a disposable diaper-type article 10 made according to a preferred embodiment of the present invention is shown. The diaper 10 includes a front waistband panel section 12, a rear waistband panel section 14, and an intermediate section 16 which interconnects the front and rear waistband sections. The diaper comprises a liquid impermeable outer cover layer 20, a liquid permeable liner layer 30, and an absorbent body 40 located between the outer cover layer and the liner layer. Fastening means, such as adhesive tapes 36 are employed to secure the diaper 10 on a wearer. The liner 30 and outer cover 20 are bonded to each other and to absorbent body 40 with lines and patterns of adhesive, such as a hot-melt, pressure-sensitive adhesive. Elastic members 60, 62, 64 and 66 can be configured about the edges of the diaper for a close fit about the wearer.

The outer cover layer 20 is composed of a liquid impermeable material such as a polymer film comprising polyethylene or polypropylene. The outer cover layer 20 may alternatively be composed of a nonwoven fibrous web constructed to provide the desired levels of liquid impermeability.

The liner layer 30 preferably comprises the permanently hydrophilic nonwoven fabric of the present invention. The absorbent body 40 may also be made of the permanently hydrophilic nonwoven fabric of the present invention. It is desirable that both the liner layer 30 and the absorbent body 40 be hydrophilic to absorb and retain aqueous fluids such as urine. Although not shown in Figure 1, the disposable diaper 10 may include additional fluid handling layers such as a surge layer, a transfer layer or a distribution layer. These layers may be separate layers or may be integral with the liner layer 20 or the absorbent pad 40. The diaper 10 may include various combinations of layers made with the permanently hydrophilic nonwoven material of the present invention and other conventional hydrophilic materials. For example, one or more of the fluid handling layers of the diaper 10 may be made of normally hydrophobic materials which have been treated to become hydrophilic and the absorbent body 40 may comprise cellulosic fibers which are naturally hydrophilic.

Although the absorbent article 10 shown in Figure 1 is a disposable diaper, it should be understood that the nonwoven fabric of the present invention may be used to make a variety of absorbent articles such as those identified above.

## Claims

1. A melt-extruded multicomponent polymeric strand including a first melt-extrudable polymeric component and a second melt-extrudable, hydrophilic polymeric component, the multicomponent strand having a cross-section, a length, and a peripheral surface, the first and second components being arranged in distinct zones across the cross-section of the multicomponent strand and extending continuously along the length of the multicomponent strand, the second component constituting at least a portion of the peripheral surface of the multicomponent strand continuously along the length of the multicomponent strand, **characterised in that** the second component has a critical surface tension at 20 ° C greater than 55 dyne/cm.

2. The melt-extruded multicomponent polymeric strand of claim 1 wherein the first and second components are arranged in a sheath/core configuration, the first component forming the core and the second component forming the sheath.

3. The melt-extruded multicomponent polymeric strand of claim 1 wherein the second component has a critical surface tension at 20 ° C greater than 65 dyne/cm.

4. The melt-extruded multicomponent polymeric strand of claim 1 wherein the second component comprises a block copolymer of nylon 6 and polyethylene oxide diamine.

5. The melt-extruded multicomponent polymeric strand of claim 1 wherein the second component comprises ethylene acrylic acid.

6. The melt-extruded multicomponent polymeric strand of claim 1 wherein the second component comprises a neutralised salt of ethylene acrylic acid.

7. The melt-extruded multicomponent polymetric strand of claim 1 wherein the first component is hydrophobic.

8. The melt-extruded multicomponent polymeric strand of claim 1 wherein the first component is selected from the group consisting of linear polycondensates and crystalline polyolefins.

9. The melt-extruded multicomponent polymeric strand of claim 1 wherein the first component comprises a polymer selected from the group consisting of polypropylene, polyethylene, copolymers of ethylene and propylene, polyethylene terephtahlate, and polyamides.

10. The melt-extruded multicomponent polymeric strand of claim 1 wherein the first component comprises a polymer selected from the group consisting of polypropylene, polyethylene, copolymers of ethylene and propylene, polyethylene terephthalate, and polyamides and the second component comprises a block copolymer of nylon 6 and polyethylene oxide diamine.

11. The melt-extruded multicomponent polymeric strand of claim 10 wherein the first and second components are arranged in a sheath/core configuration, the first component forming the core and the second component forming the sheath.

12. The melt-extruded multicomponent polymeric strand of claim 1 wherein the first component is present in an amount from 50 to 95% by weight of the strand and the second component is present in an amount from 50 to 5% of the strand.

13. The melt-extruded multicomponent polymeric strand of claim 1 wherein the first component is present in an amount from about 50 to about 85% by weight of the strand and the second component is present in an amount from about 50 to about 15% of the strand.

14. The melt-extruded multicomponent polymeric strand of claim 11 wherein the first component is present in an amount from 50 to 95% by weight of the strand and the second component is present in an amount from 50 to 5% of the strand.

15. The melt-extruded multicomponent polymeric strand of claim 11 wherein the first component is present in an amount from 50 to 85% by weight of the strand and the second component is present in an amount from 50 to 15% of the strand.

16. A non-woven fabric comprising melt-extruded multicomponent polymeric strands including a first melt-extrudable polymeric component and a second melt-extrudable, hydrophilic polymeric component, the multicomponent strands having a cross-section, a length, and a peripheral surface, the first and second components being arranged in distinct zones across the cross-section of the multicomponent strands and extending continuously along the length of the multicomponent strands, the second component constituting at least a portion of the peripheral surface of the multicomponent strands continuously along the length of the multicomponent strands, **characterised in that** the second component has a critical surface tension at 20 ° C greater than 55 dyne/cm.

17. The nonwoven fabric of claim 16 wherein the first and second components are arranged in a sheath/core configuration, the first component forming the core and the second component forming the sheath.

18. The nonwoven fabric of claim 16 wherein the second component has a critical surface tension at 20 ° C greater than 65 dyne/cm.

19. The nonwoven fabric of claim 16 wherein the second component comprises a block copolymer of nylon 6 and polyethylene oxide diamine.

20. The nonwoven fabric of claim 16 wherein the second component comprises ethylene acrylic acid.

21. The nonwoven fabric of claim 16 wherein the second component comprises a neutralised salt of ethylene acrylic acid.

22. The nowoven fabric of claim 16 wherein the first component is hydrophobic.

23. The nonwoven fabric of claim 16 wherein the first component is selected from the group consisting of linear polycondensates and crystalline polyolefins.

24. The nonwoven fabric of claim 16 wherein the first component comprises a polymer selected from the group consisting of polypropylene, polyethylene, copolymers of ethylene and propylene, polyethylene terephthalate, and polyamides.

25. The nonwoven fabric of claim 16 wherein the first component comprises a polymer selected from the group consisting of polypropylene, polyethylene, copolymers of ethylene and propylene, polyethylene terephthalate and polyamides and the second component comprises a block copolymer of nylon 6 and polyethylene oxide diamine.

26. The nonwoven fabric of claim 25 wherein the first and second components are arranged in a sheath/core configuration, the first component forming the core and the second component forming the sheath.

27. The nonwoven fabric of claim 16 wherein the first component is present in an amount from 50 to 95% by weight of the strands and the second component is present in an amount from 50 to 5% of the strands.

28. The nonwoven fabric of claim 16 wherein the first component is present in an amount from 50 to 85% by weight of the strands and the second component is present in an amount from 50 to 15% of the strands.

29. The nonwoven fabric of claim 26 wherein the first component is present in an amount from 50 to 95% by weight of the strands and the second component is present in an amount from 50 to 5% of the strands.

30. The nonwoven fabric of claim 26 wherein the first component is present in an amount from 50 to 85% by weight of the strands and the second component is present in an amount from 50 to 15% of the strands.

31. An absorbent article comprising a fluid handling layer of nonwoven fabric comprising melt-extruded multicomponent polymeric strands including a first melt-extrudable polymeric component and a second melt-extrudable, hydrophilic polymeric component, the multicomponent strands having a cross-section, a length, and a peripheral surface, the first and second components being arranged in distinct zones across the cross-section of the multicomponent strands and extending continuously along the length of the multicomponent strands, the second component constituting at least a portion of the peripheral surface of the multicomponent strands, **characterised in that** the second component has a critical surface tension at 20 ° C greater than 55 dynes/cm.

32. The absorbent article of claim 31 wherein the first and second components are arranged in a sheath/core configuration, the first component forming the core and the second component forming the sheath.

33. The absorbent article of claim 31 wherein the second component has a critical surface tension at 20 ° C greater than 65 dyne/cm.

34. The absorbent article of claim 31 wherein the second component comprises a block copolymer of nylon 6 and polyethylene oxide diamine.

35. The absorbent article of claim 31 wherein the second component comprises ethylene acrylic acid.

36. The absorbent article of claim 31 wherein the second component comprises a neutralised salt of ethylene acrylic acid.

37. The absorbent article of claim 31 wherein the first component is hydrophobic.

38. The absorbent article of claims 31 wherein the first component is selected from the group consisting of linear polycondensates and crystalline polyolefins.

39. The absorbent article of claim 31 wherein the first component comprises a polymer selected from the group consisting of polypropylene, polyethylene, copolymers of ethylene and propylene, polyethylene terephthalate, and polyamides.

40. The absorbent article of claim 31 wherein the first component comprises a polymer selected from the group consisting of polypropylene, polyethylene, copolymers of ethylene and propylene, polyethylene terephthalate, and polyamides and the second component comprises a block copolymer of nylon 6 and polyethylene oxide diamine.

41. The absorbent article of claim 40 wherein the first and second components are arranged in a sheath/core configuration, the first component forming the core and the second component forming the sheath.

42. The absorbent article of claim 31 wherein the first component is present in an amount from 50 to 95% by weight of the strands and the second component is present in an amount from 50 to 5% of the strands.

43. The absorbent article of claim 31 wherein the first component is present in an amount from 50 to 85% by weight of the strands and the second component is present in an amount from 50 to 15% of the strands.

44. The absorbent article of claim 41 wherein the first component is present in an amount form 50 to 95% by weight of the strands and the second component is present in an amount from 50 to 5% of the strands.

45. The absorbent article of claim 41 wherein the first component is present in an amount from 50 to 85% by weight of the strands and the second component is present in an amount from 50 to 15% of the strands.

46. The absorbent article of claim 31 wherein the absorbent article is an adult incontinence product.

47. The absorbent article of claim 31 wherein the absorbent article is an infant diaper.

48. The absorbent article of claim 31 wherein the absorbent article is a wipe.

49. The absorbent article of claim 31 wherein the absorbent article is a towel.

50. The absorbent article of claim 31 wherein the absorbent article is a training pant.

51. The absorbent article of claim 31 wherein the absorbent article is a feminine care absorbent product.

## Patentansprüche

1. Schmelzextrudierter, polymerer Mehrkomponentenfaden, der einen ersten schmelzextrudierbaren polymeren Bestandteil und einen zweiten schmelzextrudierbaren, hydrophilen polymeren Bestandteil einschließt, wobei der Mehrkomponentenfaden einen Querschnitt, eine Länge und eine Umfangsfläche aufweist, der erste und zweite Bestandteil in getrennten Zonen über den Querschnitt des Mehrkomponentenfadens angeordnet sind und sich entlang der Länge des Mehrkomponentenfadens ununterbrochen erstrecken und der zweite Bestandteil mindestens einen Teil der Umfangsfläche des Mehrkomponentenfadens ununterbrochen entlang der Länge des Mehrkomponentenfadens darstellt, dadurch gekennzeichnet, daß der zweite Bestandteil eine höhere kritische Oberflächenspannung als 55 dyn/cm bei 20°C aufweist.

2. Schmelzextrudierter polymerer Mehrkomponentenfaden von Anspruch 1, wobei der erste und zweite Bestandteil in einer Hülle/Kern-Anordnung angeordnet sind und der erste Bestandteil den Kern bildet und der zweite Bestandteil die Hülle bildet.

3. Schmelzextrudierter polymerer Mehrkomponentenfaden von Anspruch 1, wobei der zweite Bestandteil eine höhere kritische Oberflächenspannung als 65 dyn/cm bei 20°C aufweist.

4. Schmelzextrudierter polymerer Mehrkomponentenfaden von Anspruch 1, wobei der zweite Bestandteil ein Blockcopolymer aus Nylon 6 und Polyethylenoxiddiamin umfaßt.

5. Schmelzextrudierter polymerer Mehrkomponentenfaden von Anspruch 1, wobei der zweite Bestandteil Ethylenacrylsäure umfaßt.

6. Schmelzextrudierter polymerer Mehrkomponentenfaden von Anspruch 1, wobei der zweite Bestandteil ein neutralisiertes Ethylenacrylsäuresalz umfaßt.

7. Schmelzextrudierter polymerer Mehrkomponentenfaden von Anspruch 1, wobei der erste Bestandteil hydrophob ist.

8. Schmelzextrudierter polymerer Mehrkomponentenfaden von Anspruch 1, wobei der erste Bestandteil aus der Gruppe ausgewählt ist, die aus linearen Polykondensaten und kristallinen Polyolefinen besteht.

9. Schmelzextrudierter polymerer Mehrkomponentenfaden von Anspruch 1, wobei der erste Bestandteil ein Polymer umfaßt, das aus der Gruppe ausgewählt ist, die aus Polypropylen, Polyethylen, Copolymeren von Ethylen und Propylen, Polyethylenterephthalat und Polyamiden besteht.

10. Schmelzextrudierter polymerer Mehrkomponentenfaden von Anspruch 1, wobei der erste Bestandteil ein Polymer umfaßt, das aus der Gruppe ausgewählt ist, die aus Polypropylen, Polyethylen, Copolymeren von Ethylen und Propylen, Polyethylenterephthalat und Polyamiden besteht, und der zweite Bestandteil ein Blockcopolymer aus Nylon 6 und Polyethylenoxiddiamin umfaßt.

11. Schmelzextrudierter polymerer Mehrkomponentenfaden von Anspruch 10, wobei der erste und zweite Bestandteil in einer Hülle/Kern-Anordnung angeordnet sind und der erste Bestandteil den Kern bildet und der zweite Bestandteil die Hülle bildet.

12. Schmelzextrudierter polymerer Mehrkomponentenfaden von Anspruch 1, wobei der erste Bestandteil in einer Menge von 50 bis 95 Gew.-% des Fadens vorliegt und der zweite Bestandteil in einer Menge von 50 bis 5% des Fadens vorliegt.

13. Schmelzextrudierter polymerer Mehrkomponentenfaden von Anspruch 1, wobei der erste Bestandteil in einer Menge von etwa 50 bis etwa 85 Gew.-% des Fadens vorliegt und der zweite Bestandteil in einer Menge von etwa 50 bis etwa 15% des Fadens vorliegt.

14. Schmelzextrudierter polymerer Mehrkomponentenfaden von Anspruch 11, wobei der erste Bestandteil in einer Menge von 50 bis 95 Gew.-% des Fadens vorliegt und der zweite Bestandteil in einer Menge von 50 bis 5% des Fadens vorliegt.

15. Schmelzextrudierter polymerer Mehrkomponentenfaden von Anspruch 11, wobei der erste Bestandteil in einer Menge von 50 bis 85 Gew.-% des Fadens vorliegt und der zweite Bestandteil in einer Menge von 50 bis 15% des Fadens vorliegt.

16. Vliesstoff umfassend schmelzextrudierte, polymere Mehrkomponentenfäden, die einen ersten schmelzextrudierbaren polymeren Bestandteil und einen zweiten schmelzextrudierbaren, hydrophilen polymeren Bestandteil einschließen, wobei die Mehrkomponentenfäden einen Querschnitt, eine Länge und eine Umfangsfläche aufweisen, der erste und zweite Bestandteil in getrennten Zonen über den Querschnitt der Mehrkomponentenfäden angeordnet sind und sich entlang der Länge der Mehrkomponentenfäden ununterbrochen erstrecken und der zweite Bestandteil mindestens einen Teil der Umfangsfläche der Mehrkomponentenfäden ununterbrochen entlang der Länge der Mehrkomponentenfäden darstellt, dadurch gekennzeichnet, daß der zweite Bestandteil eine höhere kritische Oberflächenspannung als 55 dyn/cm bei 20°C aufweist.

17. Vliesstoff von Anspruch 16, wobei der erste und zweite Bestandteil in einer Hülle/Kern-Anordnung angeordnet sind und der erste Bestandteil den Kern bildet und der zweite Bestandteil die Hülle bildet.

18. Vliesstoff von Anspruch 16, wobei der zweite Bestandteil eine höhere kritische Oberflächenspannung als 65 dyn/cm bei 20°C aufweist.

19. Vliesstoff von Anspruch 16, wobei der zweite Bestandteil ein Blockcopolymer aus Nylon 6 und Polyethylenoxiddiamin umfaßt.

20. Vliesstoff von Anspruch 16, wobei der zweite Bestandteil Ethylenacrylsäure umfaßt.

21. Vliesstoff von Anspruch 16, wobei der zweite Bestandteil ein neutralisiertes Ethylenacrylsäuresalz umfaßt.

22. Vliesstoff von Anspruch 16, wobei der erste Bestandteil hydrophob ist.

23. Vliesstoff von Anspruch 16, wobei der erste Bestandteil aus der Gruppe ausgewählt ist, die aus linearen Polykondensaten und kristallinen Polyolefinen besteht.

24. Vliesstoff von Anspruch 16, wobei der erste Bestandteil ein Polymer umfaßt, das aus der Gruppe ausgewählt ist, die aus Polypropylen, Polyethylen, Copolymeren von Ethylen und Propylen, Polyethylenterephthalat und Polyamiden besteht.

25. Vliesstoff von Anspruch 16, wobei der erste Bestandteil ein Polymer umfaßt, das aus der Gruppe ausgewählt ist, die aus Polypropylen, Polyethylen, Copolymeren von Ethylen und Propylen, Polyethylenterephthalat und Polyamiden besteht, und der zweite Bestandteil ein Blockcopolymer aus Nylon 6 und Polyethylenoxiddiamin umfaßt.

26. Vliesstoff von Anspruch 25, wobei der erste und zweite Bestandteil in einer Hülle/Kern-Anordnung angeordnet sind und der erste Bestandteil den Kern bildet und der zweite Bestandteil die Hülle bildet.

27. Vliesstoff von Anspruch 16, wobei der erste Bestandteil in einer Menge von 50 bis 95 Gew.-% der Fäden vorliegt und der zweite Bestandteil in einer Menge von 50 bis 5% der Fäden vorliegt.

28. Vliesstoff von Anspruch 16, wobei der erste Bestandteil in einer Menge von 50 bis 85 Gew.-% der Fäden vorliegt und der zweite Bestandteil in einer Menge von 50 bis 15% der Fäden vorliegt.

29. Vliesstoff von Anspruch 26, wobei der erste Bestandteil in einer Menge von 50 bis 95 Gew.-% der Fäden vorliegt und der zweite Bestandteil in einer Menge von 50 bis 5% der Fäden vorliegt.

30. Vliesstoff von Anspruch 26, wobei der erste Bestandteil in einer Menge von 50 bis 85 Gew.-% der Fäden vorliegt und der zweite Bestandteil in einer Menge von 50 bis 15% der Fäden vorliegt.

31. Absorbierender Gegenstand umfassend eine flüssigkeitsleitende Schicht aus Vliesstoff, umfassend schmelzextrudierte, polymere Mehrkomponentenfäden, die einen ersten schmelzextrudierbaren polymeren Bestandteil und einen zweiten schmelzextrudierbaren, hydrophilen polymeren Bestandteil einschließen, wobei die Mehrkomponentenfäden einen Querschnitt, eine Länge und eine Umfangsfläche aufweisen, der erste und zweite Bestandteil in getrennten Zonen über den Querschnitt der Mehrkomponentenfäden angeordnet sind und sich entlang der Länge der Mehrkomponentenfäden ununterbrochen erstrecken und der zweite Bestandteil mindestens einen Teil der Umfangsfläche der Mehrkomponentenfäden darstellt, dadurch gekennzeichnet, daß der zweite Bestandteil eine höhere kritische Oberflächenspannung als 55 dyn/cm bei 20°C aufweist.

32. Absorbierender Gegenstand von Anspruch 31, wobei der erste und zweite Bestandteil in einer Hülle/Kern-Anordnung angeordnet sind und der erste Bestandteil den Kern bildet und der zweite Bestandteil die Hülle bildet.

33. Absorbierender Gegenstand von Anspruch 31, wobei der zweite Bestandteil eine höhere kritische Oberflächenspannung als 65 dyn/cm bei 20°C aufweist.

34. Absorbierender Gegenstand von Anspruch 31, wobei der zweite Bestandteil ein Blockcopolymer aus Nylon 6 und Polyethylenoxiddiamin umfaßt.

35. Absorbierender Gegenstand von Anspruch 31, wobei der zweite Bestandteil Ethylenacrylsäure umfaßt.

36. Absorbierender Gegenstand von Anspruch 31, wobei der zweite Bestandteil ein neutralisiertes Ethylenacrylsäuresalz umfaßt.

37. Absorbierender Gegenstand von Anspruch 31, wobei der erste Bestandteil hydrophob ist.

38. Absorbierender Gegenstand von Anspruch 31, wobei der erste Bestandteil aus der Gruppe ausgewählt ist, die aus linearen Polykondensaten und kristallinen Polyolefinen besteht.

39. Absorbierender Gegenstand von Anspruch 31, wobei der erste Bestandteil ein Polymer umfaßt, das aus der Gruppe ausgewählt ist, die aus Polypropylen, Polyethylen, Copolymeren von Ethylen und Propylen, Polyethylenterephthalat und Polyamiden besteht.

40. Absorbierender Gegenstand von Anspruch 31, wobei der erste Bestandteil ein Polymer umfaßt, das aus der Gruppe ausgewählt ist, die aus Polypropylen, Polyethylen, Copolymeren von Ethylen und Propylen, Polyethylenterephthalat und Polyamiden besteht, und der zweite Bestandteil ein Blockcopolymer aus Nylon 6 und Polyethylenoxiddiamin umfaßt.

41. Absorbierender Gegenstand von Anspruch 40, wobei der erste und zweite Bestandteil in einer Hülle/Kern-Anordnung angeordnet sind und der erste Bestandteil den Kern bildet und der zweite Bestandteil die Hülle bildet.

42. Absorbierender Gegenstand von Anspruch 31, wobei der erste Bestandteil in einer Menge von 50 bis 95 Gew.-% der Fäden vorliegt und der zweite Bestandteil in einer Menge von 50 bis 5% der Fäden vorliegt.

43. Absorbierender Gegenstand von Anspruch 31, wobei der erste Bestandteil in einer Menge von 50 bis 85 Gew.-% der Fäden vorliegt und der zweite Bestandteil in einer Menge von 50 bis 15% der Fäden vorliegt.

44. Absorbierender Gegenstand von Anspruch 41, wobei der erste Bestandteil in einer Menge von 50 bis 95 Gew.-% der Fäden vorliegt und der zweite Bestandteil in einer Menge von 50 bis 5% der Fäden vorliegt.

45. Absorbierender Gegenstand von Anspruch 41, wobei der erste Bestandteil in einer Menge von 50 bis 85 Gew.-% der Fäden vorliegt und der zweite Bestandteil in einer Menge von 50 bis 15% der Fäden vorliegt.

46. Absorbierender Gegenstand von Anspruch 31, wobei der absorbierende Gegenstand ein lnkontinenzprodukt für Erwachsene ist.

47. Absorbierender Gegenstand von Anspruch 31, wobei der absorbierende Gegenstand eine Säuglingswindel ist.

48. Absorbierender Gegenstand von Anspruch 31, wobei der absorbierende Gegenstand ein Wischtuch ist.

49. Absorbierender Gegenstand von Anspruch 31, wobei der absorbierende Gegenstand ein Handtuch ist.

50. Absorbierender Gegenstand von Anspruch 31, wobei der absorbierende Gegenstand eine Trainingshose ist.

51. Absorbierender Gegenstand von Anspruch 31, wobei der absorbierende Gegenstand ein absorbierendes Damenhygieneprodukt ist.

## Revendications

1. Filament polymère filé par fusion à composants multiples, comprenant un premier composant polymère pouvant être filé par fusion et un deuxième composant polymère hydrophile pouvant être filé par fusion, le filament à composants multiples ayant une section transversale, une longueur, et une surface périphérique, les premier et deuxième composants étant agencés dans des zones distinctes au travers de la section transversale du filament à composants multiples et s'étendant continûment le long de la longueur du filament à composants multiples, le deuxième composant constituant au moins une partie de la surface périphérique du filament à composants multiples continûment le long de la longueur du filament à composants multiples, caractérisé en ce que le deuxième composant a une tension superficielle critique à 20°C supérieure à 55 dynes/cm.

2. Filament polymère filé par fusion à composants multiples selon la revendication 1, dans lequel les premier et deuxième composants sont agencés selon une configuration gaine/coeur, le premier composant formant le coeur et le deuxième composant formant la gaine.

3. Filament polymère filé par fusion à composants multiples selon la revendication 1, dans lequel le deuxième composant a une tension superficielle critique à 20°C supérieure à 65 dynes/cm.

4. Filament polymère filé par fusion à composants multiples selon la revendication 1, dans lequel le deuxième composant comprend un copolymère séquencé de nylon 6 et de polyoxyéthylènediamine.

5. Filament polymère filé par fusion à composants multiples selon la revendication 1, dans lequel le deuxième composant comprend de l'éthylène/acide acrylique.

6. Filament polymère filé par fusion à composants multiples selon la revendication 1, dans lequel le deuxième composant comprend un sel neutralisé d'éthylène/acide acrylique.

7. Filament polymère filé par fusion à composants multiples selon la revendication 1, dans lequel le premier composant est hydrophobe.

8. Filament polymère filé par fusion à composants multiples selon la revendication 1, dans lequel le premier composant est choisi dans l'ensemble constitué par les produits de polycondensation linéaires et les polyoléfines cristallines.

9. Filament polymère filé par fusion à composants multiples selon la revendication 1, dans lequel le premier composant comprend un polymère choisi dans l'ensemble constitué par le polypropylène, le polyéthylène, les copolymères d'éthylène et de propylène, le poly(téréphtalate d'éthylène), et les polyamides.

10. Filament polymère filé par fusion à composants multiples selon la revendication 1, dans lequel le premier composant comprend un polymère choisi dans l'ensemble constitué par le polypropylène, le polyéthylène, les copolymères d'éthylène et de propylène, le poly(téréphtalate d'éthylène), et les polyamides, et le deuxième composant comprend un copolymère séquencé de nylon 6 et de polyoxyéthylènediamine.

11. Filament polymère filé par fusion à composants multiples selon la revendication 10, dans lequel les premier et deuxième composants sont agencés selon une configuration gaine/coeur, le premier composant formant le coeur et le deuxième composant formant la gaine.

12. Filament polymère filé par fusion à composants multiples selon la revendication 1, dans lequel le premier composant est présent en une quantité de 50 à 95 % en poids du filament et le deuxième composant est présent en une quantité de 50 à 5 % du filament.

13. Filament polymère filé par fusion à composants multiples selon la revendication 1, dans lequel le premier composant est présent en une quantité d'environ 50 à environ 85 % en poids du filament et le deuxième composant est présent en une quantité d'environ 50 à environ 15 % du filament.

14. Filament polymère filé par fusion à composants multiples selon la revendication 11, dans lequel le premier composant est présent en une quantité de 50 à 95 % en poids du filament et ie deuxième composant est présent en une quantité de 50 à 5 % du filament.

15. Filament polymère filé par fusion à composants multiples selon la revendication 11, dans lequel le premier composant est présent en une quantité de 50 à 85% en poids du filament et le deuxième composant est présent en une quantité de 50 à 15 % du filament.

16. Etoffe non-tissée comprenant des filaments polymères filés par fusion à composants multiples comprenant un premier composant polymère pouvant être filé par fusion et un deuxième composant polymère hydrophile pouvant être filé par fusion, les filaments à composants multiples ayant une section transversale, une longueur, et une surface périphérique, les premier et deuxième composants étant agencés dans des zones distinctes au travers de la section transversale des filaments à composants multiples et s'étendant continûment le long de la longueur des filaments à composants multiples, le deuxième composant constituant au moins une partie de la surface périphérique des filaments à composants multiples continûment le long de la longueur des filaments à composants multiples, caractérisée en ce que le deuxième composant a une tension superficielle critique à 20°C supérieure à 55 dynes/cm.

17. Etoffe non-tissée selon la revendication 16, dans laquelle les premier et deuxième composants sont agencés selon une configuration gaine/coeur, le premier composant formant le coeur et le deuxième composant formant la gaine.

18. Etoffe non-tissée selon la revendication 16, dans laquelle le deuxième composant a une tension superficielle critique à 20°C supérieure à 65 dynes/cm.

19. Etoffe non-tissée selon la revendication 16, dans laquelle le deuxième composant comprend un copolymère séquencé de nylon 6 et de polyoxyéthylènediamine.

20. Etoffe non-tissée selon la revendication 16, dans laquelle le deuxième composant comprend de l'éthylène/acide acrylique.

21. Etoffe non-tissée selon la revendication 16, dans laquelle le deuxième composant comprend un sel neutralisé d'éthylène/acide acrylique.

22. Etoffe non-tissée selon la revendication 16, dans laquelle le premier composant est hydrophobe.

23. Etoffe non-tissée selon la revendication 16, dans laquelle le premier composant est choisi dans l'ensemble constitué par les produits de polycondensation linéaires et les polyoléfines cristallines.

24. Etoffe non-tissée selon la revendication 16, dans laquelle le premier composant comprend un polymère choisi dans l'ensemble constitué par le polypropylène, le polyéthylène, les copolymères d'éthylène et de propylène, le poly(téréphtalate d'éthylène), et les polyamides.

25. Etoffe non-tissée selon la revendication 16, dans laquelle le premier composant comprend un polymère choisi dans l'ensemble constitué par le polypropylène, le polyéthylène, les copolymères d'éthylène et de propylène, le poly(téréphtalate d'éthylène), et les polyamides, et le deuxième composant comprend un copolymère séquencé de nylon 6 et de polyoxyéthylènediamine.

26. Etoffe non-tissée selon la revendication 25, dans laquelle les premier et deuxième composants sont agencés selon une configuration gaine/coeur, le premier composant formant le coeur et le deuxième composant formant la gaine.

27. Etoffe non-tissée selon la revendication 16, dans laquelle le premier composant est présent en une quantité de 50 à 95 % en poids des filaments et le deuxième composant est présent en une quantité de 50 à 5 % des filaments.

28. Etoffe non-tissée selon la revendication 16, dans laquelle le premier composant est présent en une quantité de 50 à 85 % en poids des filaments et le deuxième composant est présent en une quantité de 50 à 15 % des filaments.

29. Etoffe non-tissée selon la revendication 26, dans laquelle le premier composant est présent en une quantité de 50 à 95 % en poids des filaments et le deuxième composant est présent en une quantité de 50 à 5 % des filaments.

30. Etoffe non-tissée selon la revendication 26, dans laquelle le premier composant est présent en une quantité de 50 à 85 % en poids des filaments et le deuxième composant est présent en une quantité de 50 à 15 % des filaments.

31. Article absorbant comprenant une couche de traitement de fluide en étoffe non-tissée comprenant des filaments polymères filés par fusion à composants multiples comprenant un premier composant polymère pouvant être filé par fusion et un deuxième composant polymère hydrophile pouvant être filé par fusion, les filaments à composants multiples ayant une section transversale, une longueur, et une surface périphérique, les premier et deuxième composants étant agencés dans des zones distinctes au travers de la section transversale des filaments à composants multiples et s'étendant continûment le long de la longueur des filaments à composants multiples, le deuxième composant constituant au moins une partie de la surface périphérique des filaments à composants multiples, caractérisé en ce que le deuxième composant a une tension superficielle critique à 20°C supérieure à 55 dynes/cm.

32. Article absorbant selon la revendication 31, dans lequel les premier et deuxième composants sont agencés selon une configuration gaine/coeur, le premier composant formant le coeur et le deuxième composant formant la gaine.

33. Article absorbant selon la revendication 31, dans lequel le deuxième composant a une tension superficielle critique à 20°C supérieure à 65 dynes/cm.

34. Article absorbant selon la revendication 31, dans lequel le deuxième composant comprend un copolymère séquencé de nylon 6 et de polyoxyéthylènediamine.

35. Article absorbant selon la revendication 31, dans lequel le deuxième composant comprend de l'éthylène/acide acrylique.

36. Article absorbant selon la revendication 31, dans lequel le deuxième composant comprend un sel neutralisé d'éthylène/acide acrylique.

37. Article absorbant selon la revendication 31, dans lequel le premier composant est hydrophobe.

38. Article absorbant selon la revendication 31, dans lequel le premier composant est choisi dans l'ensemble constitué par les produits de polycondensation linéaires et les polyoléfines cristallines.

39. Article absorbant selon la revendication 31, dans lequel le premier composant comprend un polymère choisi dans l'ensemble constitué par le polypropylène, le polyéthylène, les copolymères d'éthylène et de propylène, le poly(téréphtalate d'éthylène), et les polyamides.

40. Article absorbant selon la revendication 31, dans lequel le premier composant comprend un polymère choisi dans l'ensemble constitué par le polypropylène, le polyéthylène, les copolymères d'éthylène et de propylène, le poly(téréphtalate d'éthylène), et les polyamides, et le deuxième composant comprend un copolymère séquencé de nylon 6 et de polyoxyéthylènediamine.

41. Article absorbant selon la revendication 40, dans lequel les premier et deuxième composants sont agencés selon une configuration gaine/coeur, le premier composant formant le coeur et le deuxième composant formant la gaine.

42. Article absorbant selon la revendication 31, dans lequel le premier composant est présent en une quantité de 50 à 95 % en poids des filaments et le deuxième composant est présent en une quantité de 50 à 5 % des filaments.

43. Article absorbant selon la revendication 31, dans lequel le premier composant est présent en une quantité de 50 à 85 % en poids des filaments et le deuxième composant est présent en une quantité de 50 à 15 % des filaments.

44. Article absorbant selon la revendication 41, dans lequel le premier composant est présent en une quantité de 50 à 95 % en poids des filaments et le deuxième composant est présent en une quantité de 50 à 5 % des filaments.

45. Article absorbant selon la revendication 41, dans lequel le premier composant est présent en une quantité de 50 à 85 % en poids des filaments et le deuxième composant est présent en une quantité de 50 à 15 % des filaments.

46. Article absorbant selon la revendication 31, dans lequel l'article absorbant est un produit destiné à l'incontinence des adultes.

47. Article absorbant selon la revendication 31, dans lequel l'article absorbant est une couche pour enfants.

48. Article absorbant selon la revendication 31, dans lequel l'article absorbant est un torchon.

49. Article absorbant selon la revendication 31, dans lequel l'article absorbant est une serviette.

50. Article absorbant selon la revendication 31, dans lequel l'article absorbant est une culotte d'apprentissage.

51. Article absorbant selon la revendication 31, dans lequel l'article absorbant est un produit absorbant destiné à l'hygiène féminine.
